# EUROPEAN PATENT APPLICATION

(11) **EP 4 717 161 A1**
(43) Date of publication of application: **01.04.2026**
(21) Application number: 25204342.7
(22) Date of filing: 24.09.2025
(51) Int. Cl.: A61B 5/07, A61B 5/00, G01R 33/09

(54) **WIRELESS COMMUNICATION CAPSULE SENSOR WITH ELECTRONIC MAGNETIC SWITCH**

(30) Priority: 27.09.2024 TW 113137126
(71) Applicant: Dotspace Inc., Wilmington, DE 19801 (US)
(72) Inventor: JOW, Ueiming, California, 92128 (US); LAI, Yen-Liang, 105010 Taipei City (TW)
(74) Representative: Karakatsanis, Georgios

(57) **Abstract**

A wireless communication capsule sensor (1) with an electronic magnetic switch (132) includes a battery (130), a microprocessor (140) and an actuation module (13). The battery (130) is electrically connected to the microprocessor (140) through an electronic magnetic switch (132) and an energy storage element (131). The actuation module (13) is electrically connected to the microprocessor (140). The energy storage element (131) stores energy using a small current outputted by the battery (130). When the microprocessor (140) is in sleep mode and the electronic magnetic switch (132) receives an external magnetic field, the electronic magnetic switch (132) outputs a trigger signal (1320) to wake up the microprocessor (140), so the microprocessor (140) uses the electrical energy of the storage element and battery (130) to drive the actuation module (13) for bio-signal detection or physical therapy. When the microprocessor (140) is in working mode and the electronic magnetic switch (132) receives the magnetic field again, the electronic magnetic switch (132) outputs a trigger signal (1320), and the microprocessor (140) stops the operation of the actuation module (13).

## Description

### FIELD OF THE INVENTION

The present disclosure relates to a bio-signal sensor, and more particularly relates to a wireless communication capsule sensor with an electronic magnetic switch.

### BACKGROUND OF THE INVENTION

Capsule sensor is designed to be administered orally to the patient, so it comes with a one-time use and disposable design. Due to the size constraints, low power electronic components and small non-rechargeable batteries are generally used. However, a small battery also means less power capacity or less current supply, which limits the usage time and standby time. There are known technologies that adopt the structure of a rechargeable or replaceable battery, however, this structure often results in an increase in the overall size and weight of the capsule and incurs a higher cost. Alternatively, there are known technologies that utilize Micro-Electro-Mechanical Systems (MEMS) reed switches as an electrical On/Off mechanism. Although this type of MEMS reed switches has the advantage of being smaller in size than the traditional mechanical reed switch, it is expensive and is not conducive to the generalization of the commercialization of the product. In this regard, the aforementioned MEMS reed switch uses a magnetic reed as the main body to convert mechanical actions into electronic signals, and generally consists of a magnet on a side and a reed sensor on the other side. The MEMS reed switch can be used for vertical or horizontal sensing, and the horizontal part of the sensing can generate a single or three impulses to realize the debug function in order to avoid failure and malfunction. The so-called reed switch is made of two low-magnetic hysteresis ferrous reeds placed in parallel and completely sealed in a glass tube filled with inert gas, and small parts of the tails of the two overlap with each other to form a gap; when there is a magnetic field nearby, the overlap of the two reeds will be sensed at the opposite polarity of the magnetism, which is large enough to be attracted to define a contact. The reed switch has no mechanical parts, so there will be no issues of plugging, jamming, etc. This almost barrier-free operation can last for millions of times at high speed and with high precision. Therefore, moving the permanent magnet close to the reed switch will trigger the sensor to switch when the magnetic field is present or cut off. However, due to the expensive price, the reed switch is difficult to be popular and accepted by the market, therefore, it is a subject for related manufacturers to find a way of optimizing power management, using electronic switch to replace mechanical switch in order to reduce the occurrence of power-on errors caused by accidental touch of the switch, overcoming the above-mentioned shortcomings, and further improving the overall sealing and waterproof structure.

### SUMMARY OF THE INVENTION

The purpose of the present invention is to provide a wireless communication capsule sensor that uses an electronic magnetic switch to replace a physical control switch, so as to avoid power loss caused by accidentally touching the power supply during transportation, and ensure that the capsule has a sufficient service life and quality when it is put into a living body for bio-signal detection or physical therapy.

To achieve the aforementioned objective, the present disclosure discloses a wireless communication capsule sensor with an electronic magnetic switch, which includes a battery, a microprocessor, a wireless transmission circuit, an antenna and an actuation module, the battery is electrically connected to the microprocessor and the actuation module, the actuation module is electrically connected to the microprocessor, and the wireless transmission circuit is electrically connected to the microprocessor and the antenna, characterized in that an electronic magnetic switch and an energy storage element are connected between the battery and the microprocessor, the energy storage element uses the small current outputted by the battery to store energy. When the microprocessor is in a sleep mode and the electronic magnetic switch receives the magnetic field applied from the outside, the electronic magnetic switch outputs a trigger signal to wake up the microprocessor and enter a working mode, so that the microprocessor uses the energy storage element and the battery to provide electrical energy to drive the actuation module to perform bio-signal detection or physical therapy. Moreover, when the microprocessor is in the working mode and the electronic magnetic switch receives the magnetic field applied from the outside again, the electronic magnetic switch outputs a trigger signal again, so that the microprocessor enters the sleep mode and stops the actuation module. By the way, since the electronic magnetic switch is directly connected to the battery and is always in a low current working mode, for example, after being triggered to enter the working mode, the microprocessor works at 150nA, and after being triggered to enter the sleep mode, the microprocessor works at 100nA. Therefore, the electronic magnetic switch will output a trigger signal to the microprocessor at the moment of sensing the magnetic field, and the microprocessor connected to the battery and implanted with latch instructions will be triggered to repeatedly toggle between the sleep and the working mode.

The technical characteristics of the present invention will become apparent with the detailed description of preferred embodiments accompanied with the illustration of related drawings.

### BRIEF DESCRIPTION OF THE DRAWINGS

FIG. 1 is a schematic block diagram of a first embodiment of the present disclosure;
FIG. 2 is an exploded view of a second embodiment of the present disclosure;
FIG. 3 is a cross-sectional view of the second embodiment of the present disclosure;
FIG. 4 is an exploded view of a third embodiment of the present disclosure;
FIG. 5 is a schematic view showing some of the assemblies of the third embodiment of the present disclosure; and
FIG. 6 is a cross-sectional view of a third embodiment of the present disclosure.

### DESCRIPTION OF THE PREFERRED EMBODIMENTS

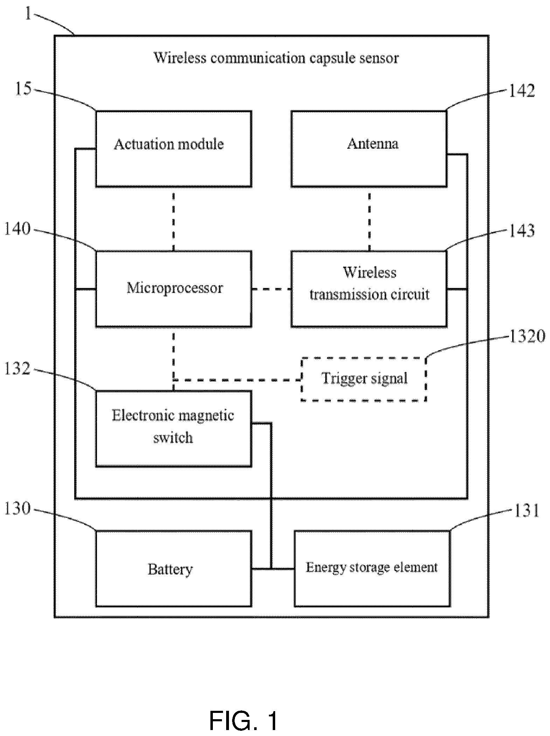

With reference to FIG. 1 for the schematic block diagram of the first embodiment of the present disclosure. A wireless communication capsule sensor 1 with an electronic magnetic switch 132 is provided with a battery 130, a microprocessor 140, a wireless transmission circuit 143, an antenna 142 and an actuation module 15, the actuation module 15 is electrically connected to the microprocessor 140, the microprocessor 140 and the actuation module 15 are electrically connected to the battery 130, an electronic magnetic switch 132 and an energy storage element 131 are connected between the battery 130 and the microprocessor 140, the energy storage element 131 uses a small current outputted by the battery 130 to store energy, and the microprocessor 140 is electrically connected to the wireless transmission circuit 143 and the antenna 142.

While the microprocessor 140 is in the sleep mode and the electronic magnetic switch 132 receives an externally applied magnetic field, the electronic magnetic switch 132 outputs a trigger signal 1320 to wake up the microprocessor 140 to enter the working mode, so that the microprocessor 140 uses the energy storage element 131 and the battery 130 to provide electrical energy to drive the actuation module 15 to perform bio-signal detection or physical therapy. Furthermore, while the microprocessor 140 is in the working mode and the electronic magnetic switch 132 receives the externally applied magnetic field again, the electronic magnetic switch 132 outputs a trigger signal 1320 to the microprocessor 140 again, so that the microprocessor 140 stops the operation of the actuation module 15. It is noteworthy that the microprocessor 140 implanted with latch instructions will be triggered to repeatedly toggle between the sleep and the working mode.

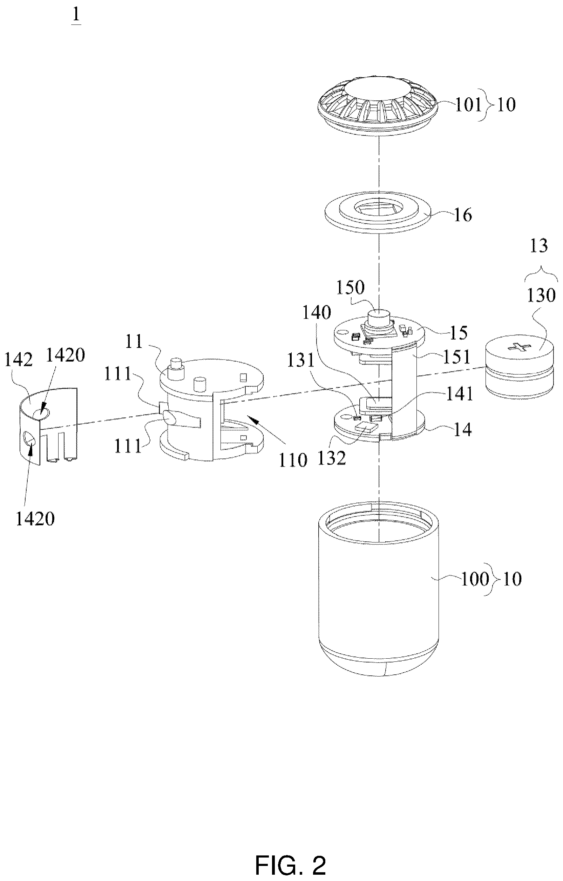

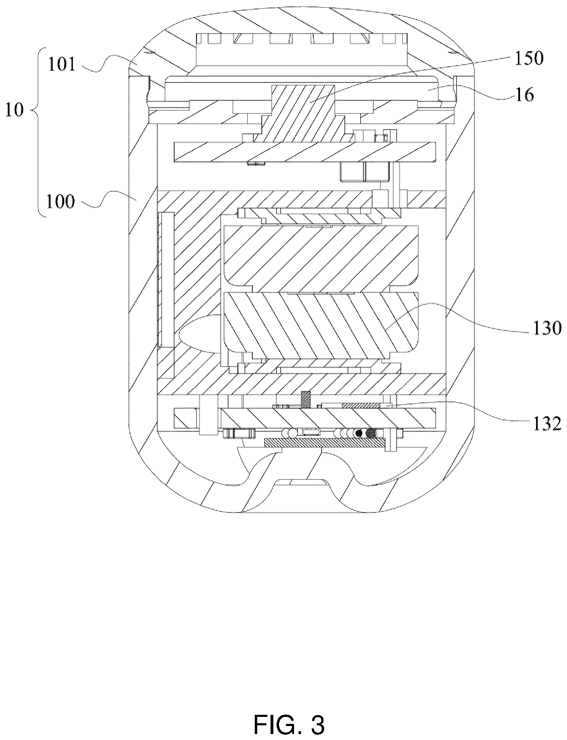

With reference to FIGS. 2 and 3 for the exploded view and cross-sectional view of the second embodiment of the present disclosure, the wireless communication capsule sensor 1 includes a capsule-type shell 10, an assembly frame 11, an energy module 13, a control module 14 and an actuation module 15, the energy module 13 is electrically connected to the control module 14 and the actuation module 15, and the actuation module 15 is electrically connected to the control module 14. The energy module 13 comprises a battery 130, an energy storage element 131, an electronic magnetic switch 132 and an electrode metal sheet, and the control module 14 includes a microprocessor 140, a quartz oscillator 141, a wireless transmission circuit (not shown in the figure) and an antenna 142. The quartz oscillator 141 is electrically connected to the microprocessor 140 for ensuring the stability and accuracy of the microprocessor 140, the antenna 142 is an antenna flexible circuit and electrically connected to the microprocessor 140, the wireless transmission circuit and the antenna 142, and provided for the control module 14 to telecommunicatively connect to an external monitoring system (not shown in the figure). In addition, the microprocessor 140, the quartz oscillator 141, the wireless transmission circuit, the energy storage element 131 and the electronic magnetic switch 132 can be installed on the circuit board of the control module 14 without restrictions.

The capsule-type shell 10 is provided with a cylinder container 100 and a cap 101, and the cylinder container 100 is dispensed with glue and assembled with the cap 101 to form a sealing structure. The assembly frame 11 is approximately cylindrical in shape, one side of the assembly frame 11 is formed with an accommodation slot 110 for accommodating the battery 130 and the electrode metal sheet, the other side of the assembly frame 11 is provided and surrounded with the antenna 142, and the antenna 142 is configured to be opposite to the accommodation slot 110. The top side of the assembly frame 11 is assembled with a circuit board and an actuation element 150 of the actuation module 15, and the bottom side of the assembly frame 11 is assembled with the circuit board of the control module 14. A data cable 151 is provided between the circuit board of the actuation module 15 and the circuit board of the control module 14 for data transmission. In this way, the assembly frame 11 is assembled with the energy module 13, the control module 14 and the actuation module 15, so that these components are stably placed in the sealed structure. In this embodiment, the side of the assembly frame 11 surrounded with the antenna 142 is provided with a plurality of protruding columns 111 in various shapes or different non-uniform shapes, the antenna 142 is provided with a plurality of holes 1420, the antenna 142 corresponds to each protruding column 111 and has a hollow design with a plurality of holes 1420 in a variety of different shapes. By engaging the protruding columns 111 with the holes 1420, the connection stability between the assembly frame 11 and the antenna 142 is improved to prevent loosening or slipping to cause signal transmission errors or interruptions. Where the actuation element 150 of the actuation module 15 can be a pressure sensor, a temperature sensor or a pH sensor, where the pressure sensor can be a piezoelectric pressure sensor, a piezoresistive pressure sensor, a capacitive pressure sensor, an electromagnetic pressure sensor or a vibrating wire pressure sensor. The piezoelectric pressure sensor converts the measured pressure into electric energy by the piezoelectric effect, and it is made of a piezoelectric material such as sodium dihydrogen phosphate, sodium potassium tartrate or quartz, etc. and while an external force is acted on the piezoelectric material, electric charges are formed on the surface of the piezoelectric material, and after the charges are amplified, the sensed pressure is converted into a proportional electrical output.

The piezoresistive pressure sensor uses the piezoresistive effect to change the resistance of a material under stress. Unlike the piezoelectric effect, the piezoresistive effect only produces impedance changes and does not generate charges. The piezoresistive effect in semiconductor materials is much greater than that in metals. Silicon can be used as the main material. The piezoresistive pressure sensor is generally connected to a Wheatstone bridge via leads. While no pressure is applied to the sensitive core, the electric bridge is in a balanced state. While the sensor is under pressure, the chip resistance changes and the electric bridge loses balance. While a constant current or constant voltage power source is added to the electric bridge, the electric bridge will output a voltage signal corresponding to the pressure and use the resistance change of the sensor to convert the voltage signal into a pressure signal output by the electric bridge. Therefore, the change in the resistance value detected by the electric bridge is amplified, and then converted into a corresponding current signal through voltage-current conversion.

The working principle of the electromagnetic pressure sensor, including the inductive pressure sensor, the Hall pressure sensor, and the eddy current pressure sensor, is described below. The inductive pressure sensor is made of various magnetic materials with different magnetic permeabilities. While pressure acts on the membrane of the sensor, the size of the air gap changes. The change in the air gap affects the change in the inductance of the coil and converts the change in inductance into a corresponding signal output to achieve the purpose of measuring pressure. The Hall pressure sensor is made of semiconductor materials based on the Hall effect. The Hall effect refers to placing a solid conductor in a magnetic field and passing a current through the solid conductor. Under the action of the Lorentz force, the current in the conductor deflects to a side and generates a voltage (Hall voltage). The electric field force caused by the voltage will balance the Lorentz force. Through the polarity of the Hall voltage, it can be confirmed that the current inside the conductor is caused by the movement of negatively charged particles (free electrons). The eddy pressure sensor is a pressure sensor with an eddy current effect, which is caused by the intersection of moving magnetic field and metal conductor, or caused by the perpendicular intersection of moving metal conductor and magnetic field.

The capacitive pressure sensor is a pressure sensor that converts the measured pressure into a change in capacitance, and it can use a round metal film or metal plating as the electrode of the capacitor. While the film is deformed under pressure, the capacitance formed between the film and the fixed electrode changes. The electrical signal output by the measuring circuit has a corresponding relationship with the voltage. The vibrating wire pressure sensor is a sensor that uses time and frequency signals to sense pressure.

The temperature sensor is a contact temperature sensor, which can be a thermistor or a resistance thermometer. The pH value sensor can be a sensor made of a polymer material or a special metal coated by a micro-electromechanical process, and the reaction of such special metal is used to detect the pH value.

An electronic magnetic switch 132 and an energy storage element 131 are connected between the battery 130 and the microprocessor 140. In some embodiments, the energy storage element 131 has a total capacitance of luF~1mF and includes at least a capacitor to utilize the small current outputted by the battery 130 to store energy. The electronic magnetic switch 132 may be a tunnel magnetoresistance (TMR) switch, which utilizes the magnetic tunnel effect to change the resistance value and output a trigger signal (not shown in the figure). Initially, the electronic magnetic switch 132 is in an OFF state. While the microprocessor 140 is in the sleep mode and the electronic magnetic switch 132 receives an externally applied magnetic field, the electronic magnetic switch 132 outputs a trigger signal to wake up the microprocessor 140, so that the microprocessor 140 uses the energy storage element 131 and the battery 130 to provide electrical energy to drive the actuation element 150 to perform bio-signal detection or physical therapy. While the microprocessor 140 is in the working mode and the electronic magnetic switch 132 receives the externally applied magnetic field again, the electronic magnetic switch 132 outputs a trigger signal to the microprocessor 140 again. At this time, the microprocessor 140 enters the sleep mode again and stops the actuation module 15. It is noteworthy that the microprocessor 140 implanted with latch instructions will be triggered to repeatedly toggle between the sleep and the working mode, and when the microprocessor 140 is in the sleep mode, the microprocessor 140 is maintained in a standby (or shutdown) state with an operating current of 1fA~100uA.

Since the cap 101 has a plurality of filter holes and provides a cover body that can filter impurities and has waterproof and breathable effects, and allows the actuation element 150 to operate in the body without being disturbed by biological tissue fluid, it is necessary to further ensure the waterproof and breathable effect to make sure that the actuation element 150 can operate without doubt. Therefore, the wireless communication capsule sensor 1 may be further provided with a filter membrane 16, which is sandwiched between the cap 101 and the assembly frame 11 and covered on the actuation module 15 to form another layer of waterproof and breathable protective membrane. Alternatively, the filter membrane 16 is attached to the top surface of the assembly frame 11 by dispensing glue. In order to prevent the glue from spilling and contaminating the actuation module 15, the top surface of the assembly frame 11 may be concavely provided with at least a dispensing groove (not shown in the figure) for receiving the glue and dispensing glue to adhere the filter membrane 16 to the top surface of the assembly frame 11. The filter membrane 16 may be donut-shaped and an opening is opened at the center of the circular sheet (as shown in Figures 2 and 3). The opening accommodates the actuation element 150, so that the filter membrane 16 is attached to the actuation module 15. At this time, the periphery of the opening may be glued to form a sealing rubber ring to enhance the waterproofness and improve the waterproofness of the structure. In this way, a wireless communication capsule sensor 1 with high waterproof effect is formed.

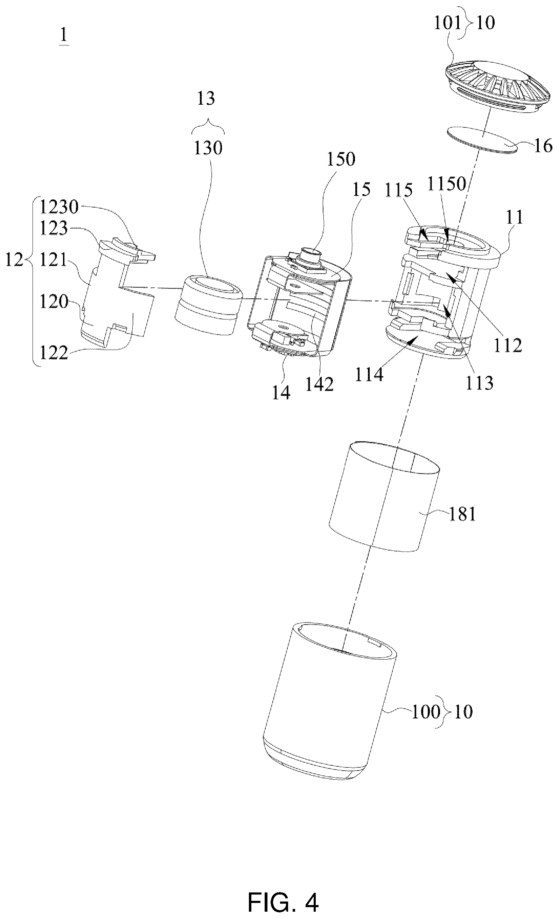

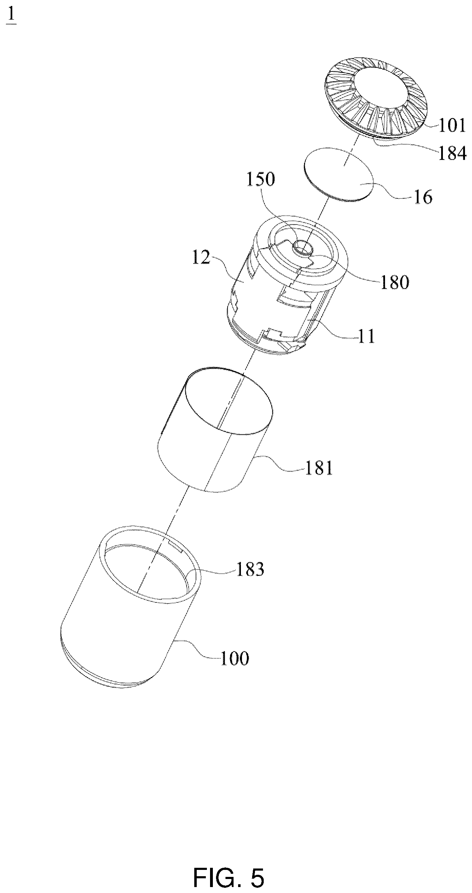

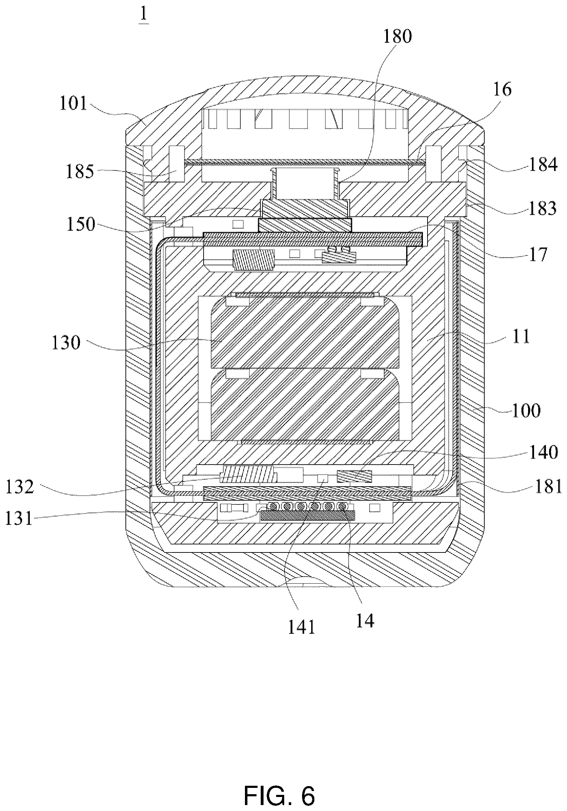

With reference to FIGS. 4 to 6 for the exploded view, the partial assembly view and the cross-sectional view of the third embodiment of the present disclosure respectively, the wireless communication capsule sensor 1 is telecommunicatively connected to an external monitoring system (not shown in the figure) and includes a capsule-type shell 10, an assembly frame 11, an assembly part 12, an energy module 13, a control module 14 and an actuation module 15. The capsule-type shell 10 is made of a biocompatible material including polycarbonate (PC) and is provided with a cylinder container 100 and a cap 101. The cylinder container 100 is glued and assembled with the cap 101 to form a sealing structure. Furthermore, the assembly frame 11 and the assembly part 12 may be made of a non-biocompatible material including polyamide (PA) and a hardening material, and the hardening material includes a fiber material accounting for 10~40% of the total amount of manufacturing materials. The assembly frame 11 is approximately cylindrical in shape, and is provided with a first compartment 112, a second compartment 113 and a third compartment 114 in the order from top to bottom, the first compartment 112 contains a circuit board of the actuation module 15, the second compartment 113 contains a battery 130 and an electrode metal sheet of the energy module 13, the third compartment 114 contains a circuit board of the control module 14, the circuit board of the actuation module 15 and the circuit board of the control module 14 are connected through an antenna 142 of the control module 14, the antenna 142 can be an antenna flexible circuit and surrounded around a sidewall of the assembly frame 11. The other sidewall of the assembly frame 11 is configured to be opposite to the antenna 142, the other sidewall of the assembly frame 11 is a hollow wall for assembling the assembly part 12, the assembly frame 11 is concavely formed with an assembly slot 115, the assembly slot 115 is assembled to the top surface of a side of the assembly part 12, and the front end of the assembly slot 115 is provided with a second concave arc 1150.

The assembly part 12 is provided with a first part 120. The first part 120 corresponds to the hollow wall structure of the assembly frame 11. The first part 120 may be in the shape of a curved sheet and its left and right sides extend forward in a curved shape to form a second part 121 and a third part 122 respectively. The areas of the second part 121 and the third part 122 are both smaller than the first part 120, so that the assembly part 12 forms a shape slightly like a cross. A fourth part 123 is disposed above the first part 120. The fourth part 123 corresponds to the assembly slot 115 structure and extends vertically forward. A first concave arc 1230 is disposed at the front end of the fourth part 123 corresponding to the second concave arc 1150. When the assembly part 12 is assembled to the assembly frame 11, the fourth part 123 is assembled to the assembly slot 115, and the first concave arc 1230 is joined to the second concave arc 1150 to form a circular hole for accommodating an actuation element 150 of the actuation module 15. In this way, the energy module 13, the control module 14 and the actuation module 15 are stably installed between the assembly frame 11 and the assembly part 12 to form a semi-assembled product, ready to be placed in the sealed structure. Wherein, part or all of the circuit elements of the control module 14 and the actuation module 15 are coated with insulating materials. Except for the top of the actuation element 150 that will contact the human body, the circuit board surface of the actuation module 15 and even part or all of the circuit board and wiring surface of the control module 14 are coated with an insulating material, such as non-biocompatible conformal coating, to form an insulating layer 17, so as to improve the insulation of the overall structure and improve the service life and product quality.

The energy module 13 is electrically connected to the control module 14 and the actuation module 15, and the actuation module 15 is telecommunicatively connected to the control module 14. The energy module 13 is provided with a battery 130, an energy storage element 131, an electronic magnetic switch 132 and an electrode metal sheet, the control module 14 is provided with a microprocessor 140, a quartz oscillator 141, a wireless transmission circuit (not shown in the figure) and antenna 142, and the quartz oscillator 141 is electrically connected to the microprocessor 140 to ensure the stability and accuracy of microprocessor 140. The wireless transmission circuit is electrically connected to the microprocessor 140 and the antenna 142 and provided for the control module 14 to telecommunicatively connect to an external monitoring system (not shown in the figure). The microprocessor 140, the quartz oscillator 141, the wireless transmission circuit, the energy storage element 131 and the electronic magnetic switch 132 can all be installed on the circuit board of the control module 14 without restrictions, and the energy storage element 131 and the electronic magnetic switch 132 are installed between the battery 130 and the microprocessor 140, and the energy storage element 131 has a total capacitance of 1uF~1mF and includes at least a capacitor to store energy using the small current outputted by the battery 130. By the way, the total capacitance of the capacitor of the energy storage element 131 depends on the circuit operation requirements, that is, if the wireless communication capsule sensor 1 requires fast wireless transmission, a larger capacitance is required because of the need to quickly draw current, otherwise a larger capacitance is not required. The electronic magnetic switch 132 may be a tunnel magnetoresistance (TMR) switch, which utilizes the magnetic tunnel effect to change the resistance value and output a trigger signal.

When the microprocessor 140 is in the sleep mode and the electronic magnetic switch 132 receives an externally applied magnetic field, the electronic magnetic switch 132 outputs a trigger signal 1320 to wake up the microprocessor 140, so that the microprocessor 140 uses the energy storage element 131 and the battery 130 to provide power to drive the actuation element 150 to perform bio-signal detection or physical therapy and feedback an actuation result, and the control module 14 transmits the actuation result to the monitoring system for remote monitoring. When the microprocessor 140 is in the working mode and the electronic magnetic switch 132 receives the externally applied magnetic field again, the electronic magnetic switch 132 outputs a trigger signal 1320 to the microprocessor 140 again. At this time, the microprocessor 140 enters the sleep mode again and stops the operation of the actuation module 15. It is noteworthy that the microprocessor 140 implanted with latch instructions will be triggered to repeatedly toggle between the sleep and the working mode, and when the microprocessor 140 enters the sleep mode, the microprocessor 140 is maintained in a standby (or shutdown) state with an operating current of 1fA~100uA.

In this embodiment, when the wireless communication capsule sensor 1 is assembled by dispensing glue, in order to avoid leakage of glue material during dispensing and thus loss of structural life due to leakage current, after the circular hole accommodates the actuation element 150, the periphery of the circular hole is dispensed with glue to form a first sealing rubber ring 180 to close the assembly gap, and the assembly part 12 and the assembly frame 11 are also dispensed with glue along the joint track to close the joint gap, so as to improve the waterproofness of the overall structure, or, the surface of the assembly frame 11 after being assembled with the assembly part 12 is dispensed with glue using biocompatible epoxy resin to further improve the insulation of the structure. After the assembly part 12 and the assembly frame 11 are connected, a solder mask tape 181 may be placed around the periphery of the semi-assembled product to further enhance the insulation protection of the main circuit components in the structure and prevent leakage current from damaging the components when the glue material leaks during the subsequent assembly dispensing process. The inner side of the opening end of the cylinder container 100 may be provided with a glue overflow groove 183 for placing the semi-assembled product into the sealing structure. When assembling, glue is continuously dispensed on the cylinder container 100 and the cap 101 to accommodate excess glue to prevent current leakage. The wireless communication capsule sensor 1 is further provided with an O-ring 184, which is mounted on the cap 101 and sandwiched between the cap 101 and the cylinder container 100 to enhance the sealing strength of the connection between the cap 101 and the cylinder container 100, thereby effectively preventing the infiltration of biological tissue fluid or water vapor. Through the design of the overflow grooves and dispensing grooves, in addition to accommodating the sealing material during the dispensing operation, the structural characteristics of the grooves can also be used to prevent excess sealing material from escaping and contaminating circuit components, thereby effectively preventing damage to components caused by current leakage, and ensuring the lifespan and quality of the wireless communication capsule sensor.

In addition, since the cap 101 has a plurality of filter holes and provides a cover body that filters impurities and has waterproof and breathable effects, the actuation element 150 is allowed to actuate in the body without being disturbed by the biological tissue fluid. In order to ensure the correct operation of the actuation element 150, it is necessary to further ensure the waterproof and breathable effect between the actuation element 150 and the cap 101. Therefore, the wireless communication capsule sensor structure 1 may be further provided with a filter membrane 16, which is sandwiched between the assembly part 12 and the assembly frame 11, or sandwiched between the cap 101, the assembly part 12 and the assembly frame 11. The filter membrane 16 is covered on the actuation module 15 to form another layer of waterproof and breathable protective film. Alternatively, the filter membrane 16 is adhered to the top surfaces of the assembly part 12 and the assembly frame 11 by dispensing glue. In order to prevent the glue from spilling and contaminating the actuation module 15, at least a dispensing groove 185 is concavely provided on the top surfaces of the assembly part 12 and the assembly frame 11 for receiving the glue to dispense glue and adhere the filter membrane 16 to the top surface of the assembly frame 11. The filter membrane 16 may be in a donut shape and an opening is provided at the center of the circular sheet (as shown in FIGS. 2 and 3). The opening accommodates the actuation element 150, so that the filter membrane 16 is attached to the actuation module 15. The periphery of the opening is glued to form a second sealing rubber ring to improve the waterproofness. The periphery of the donut-shaped filter membrane 16 and the opening of the cylinder container 100 can also be glued to improve the seal strength of the assembly and form a waterproof structure, thus forming a wireless communication capsule sensor 1 with high waterproofness.

In summation of the description above, the present disclosure utilizes an electronic magnetic switch to replace a conventional switch, and constructs an energy storage element to store energy in order to provide a sufficient amount of electrical energy for the capsule sensor when it is working, thereby ensuring that the wireless communication capsule sensor is sufficiently actuated and achieving the purpose of reducing the capsule volume. Furthermore, the overall structure has the functions of high sealing strength and high waterproofness through the following technical features, thereby ensuring the long service life, high precision actuation and high quality stability of the wireless communication capsule sensor:

1. The energy stored by the capacitor of the energy storage element can provide high current. Although the battery has high energy, it cannot provide high current. In applications, the wireless transmission circuit needs high current to work when transmitting wireless signals. Therefore, the capacitor is responsible for providing instantaneous current to meet the wireless communication needs of the wireless communication capsule sensor. Furthermore, the capsule sensor also needs a large current to work in the moment when performing certain physical therapy mechanisms of electrical stimulation. The market adaptability of the present disclosure can be further improved by setting a capacitor with an adaptive capacitance.

2. By the arrangement of the assembly frame with a hollow side, and the first compartment, the second compartment and the third compartment, the electrode metal sheet of the energy module can be separated from the circuit components above and below, thereby preventing the circuit components from being damaged when leakage current occurs.

3. By forming the circular hole by the first concave arc of the assembly part and the second concave arc of the assembly frame, it allows the actuation element with higher tolerance to protrude from the top surface of the assembly frame which is connected with the assembly part and separated from the circuit elements below, thereby further preventing the circuit elements from being affected by moisture and causing damage due to short circuit.

4. By dispensing glue to the opening of the filter membrane, the round hole on the assembly frame and along the assembly track of the assembly part and the assembly frame, the system effectively utilizes the sealing material to form a sealing rubber ring or a sealing strip to greatly improve the waterproofness of the overall structure.

5. By using the modular assembly frame and the assembly part in combination with the capsule-type shell, the sealing direction of the overall structure is oriented at the sensor, that is, around the actuation element rather than on the sidewall of the wireless communication capsule sensor. This design reduces the cumbersome assembly of the conventional side-fitting method, increases the bite force between structural components, improves the overall structural bonding strength, and effectively prevents the looseness of circuit components that will affect the sensor actuation quality.

## Claims

1. A wireless communication capsule sensor (1) with an electronic magnetic switch (132), comprising a battery (130), a microprocessor (140), a wireless transmission circuit (143), an antenna (142) and an actuation module (13), the battery (130) being electrically coupled to the microprocessor (140) and the actuation module (13), the actuation module (13) being electrically coupled to the microprocessor (140), and the wireless transmission circuit (143) being electrically coupled to the microprocessor (140) and the antenna (142), **characterized in that** an electronic magnetic switch (132) and an energy storage element (131) are connected between the battery (130) and the microprocessor (140), the energy storage element (131) stores energy using a small current outputted by the battery (130); when the microprocessor (140) is in a sleep mode and the electronic magnetic switch (132) receives an externally applied magnetic field, the electronic magnetic switch (132) outputs a trigger signal (1320) to wake up the microprocessor (140) to enter a working mode, so that the microprocessor (140) uses the electrical energy supplied by the energy storage element (131) and the battery (130) to drive the actuation module (13) to perform a bio-signal detection or a physical therapy; and when the microprocessor (140) is in the working mode and the electronic magnetic switch (132) receives the externally applied magnetic field again, the electronic magnetic switch (132) outputs a trigger signal (1320) again, so the microprocessor (140) stops the operation of the actuation module (13).

2. The wireless communication capsule sensor (1) with an electronic magnetic switch (132) according to claim 1, wherein the electronic magnetic switch (132) is a tunnel magnetoresistance (TMR) switch, which utilizes a magnetic tunnel effect to change a resistance value and output each of the trigger signals (1320).

3. The wireless communication capsule sensor (1) with an electronic magnetic switch (132) according to claim 1, wherein the microprocessor (140) is implanted with latch instructions and is triggered to repeatedly toggle between the sleep and the working mode.

4. The wireless communication capsule sensor (1) with an electronic magnetic switch (132) according to claim 1, wherein the energy storage element (131) comprises at least one capacitor.

5. The wireless communication capsule sensor (1) with an electronic magnetic switch (132) according to claim 1, further comprising a control module (14), provided with the microprocessor (140), the wireless transmission circuit (143), the antenna (142) and a quartz oscillator (141), the quartz oscillator (141) is provided for ensuring the stability and accuracy of the microprocessor (140), and when the microprocessor (140) is in the sleep mode, the microprocessor (140) is maintained in a standby or shutdown state with an operating current of 1fA~100uA.

6. The wireless communication capsule sensor (1) with an electronic magnetic switch (132) according to claim 5, further comprising a capsule-type shell (10) and an assembly frame (11), and the antenna (142) being an antenna flexible circuit and telecommunicatively coupled to an external monitoring system; a side of the assembly frame (11) being formed with an accommodation slot (110), the top side of the assembly frame (11) being installed with a circuit of the actuation module (13), the bottom side of the assembly frame (11) being installed with a circuit board of the control module (14), the accommodation slot (110) accommodating the battery (130), the other side of the assembly frame (11) surrounding the antenna (142), and the antenna (142) corresponding to the accommodation slot (110), such that the actuation module (13), the battery (130) and the control module (14) are stably installed in the capsule-type shell (10); and when the actuation module (13) performs a bio-signal detection or a physical therapy, an operation result being fed back, and the control module (14) transmitting the operation result to the monitoring system for remote monitoring.

7. The wireless communication capsule sensor (1) with an electronic magnetic switch (132) according to claim 6, wherein the same side of the assembly frame (11) surrounding the antenna (142) is provided with a plurality of protruding columns (111), the antenna (142) is provided with a plurality of holes (1420), and the protruding columns (111) are coupled to the holes (1420) to improve the connection stability between the assembly frame (11) and the antenna (142).

8. The wireless communication capsule sensor (1) with an electronic magnetic switch (132) according to claim 5, further comprising a capsule-type shell (10), an assembly frame (11) and an assembly part (12), the antenna (142) being an antenna flexible circuit and telecommunicatively coupled to an external monitoring system; the assembly frame (11) being approximately cylindrical in shape and sequentially comprising a first compartment (112), a second compartment (113) and a third compartment (114) in the order from top to bottom, the first compartment (112) accommodating a circuit board of the actuation module (13), the second compartment (113) accommodating the battery (130), the third compartment (114) accommodating a circuit board of the control module (14), the antenna (142) surrounding and covering a sidewall of the assembly frame (11), another sidewall of the assembly frame (11) being configured to be opposite to the antenna (142), the other sidewall of the assembly frame (11) being hollow for assembling the assembly part (12), so that the actuation module (13), the battery (130) and the control module (14) are stably installed in the capsule-type shell (10); when the actuation module (13) performs a bio-signal detection or a physical therapy, an operation result being fed back, and the control module (14) transmitting the operation result to the monitoring system for remote monitoring.

9. The wireless communication capsule sensor (1) with an electronic magnetic switch (132) according to claim 8, wherein, the assembly part (12) is provided with a first part (120), the left and right sides of the first part (120) extend forward with an arc to form a second part (121) and a third part (122), and the areas of the second part (121) and the third part (122) are smaller than the first part (120), the upper side of the first part (120) extends vertically forward to form a fourth part (123), and the front end of the fourth part (123) is provided with a first concave arc (1230); the assembly frame (11) is concavely formed with an assembly slot (115), the assembly slot (115) corresponds to the fourth part (123) on the top side of the assembly part (12), and the front end of the assembly slot (115) corresponds to the first concave arc (1230) and is provided with a second concave arc (1150), and when the assembly part (12) is assembled to the assembly frame (11) and the fourth part (123) is assembled to the assembly slot (115), the first concave arc (1230) is coupled to the second concave arc (1150) to form a circular hole for accommodating an actuation element (150) of the actuation module (13).

10. The wireless communication capsule sensor (1) with an electronic magnetic switch (132) according to claim 9, further comprising a filter membrane (16) provided for covering the actuation module (13) to prevent biological tissue fluids or moisture from penetrating and damaging the actuation element (150); and the top surfaces of the assembly part (12) and the assembly frame (11) being concavely provided with at least one glue dispensing groove (185), so as to prevent the glue from spilling and contaminating the actuation module (13) when the filter membrane (16) is glued to the top surfaces of the assembly part (12) and the assembly frame (11).

11. The wireless communication capsule sensor (1) with an electronic magnetic switch (132) according to claim 10, wherein the filter membrane (16) is donut-shaped and has an opening in the center of the circular sheet, the opening accommodates the actuation element (150), so that the filter membrane (16) is attached to the actuation module (13), and the periphery of the opening is glued to form a sealing rubber ring (180) to improve the waterproofness.

12. The wireless communication capsule sensor (1) with an electronic magnetic switch (132) according to claim 11, wherein the capsule-type shell (10) is made of a biocompatible material containing polycarbonate, and the assembly frame (11) and the assembly part (12) are made of a non-biocompatible material containing polyamide and a hardening material, and the hardening material contains a fiber material accounting for 10~40% of the total amount of the manufacturing materials.
